# EUROPEAN PATENT SPECIFICATION

(11) **EP 2 735 309 B1**
(45) Date of publication and mention of the grant of the patent: **27.07.2016**
(21) Application number: 12382463.3
(22) Date of filing: 27.11.2012
(51) Int. Cl.: A61K 31/19, A61K 9/00, A61K 9/06, A61K 45/06, A61K 47/12, A61K 47/18, A61P 19/04

(54) **Pharmaceutical composition for the treatment of calcific tendinitis and/or calcific bursitis**
Pharmazeutische Zusammensetzung zur Behandlung von kalzifizierender Tendinitis und/oder kalzifizierender Bursitis
Composition pharmaceutique pour le traitement de la tendinite calcifiée et/ou la bursite calcifiée

(43) Date of publication of application: 28.05.2014
(73) Proprietor: Cos Alfonso, Eduardo, 08304 Mataro (ES)
(72) Inventor: Cos Alfonso, Eduardo, 08304 Mataro (ES)
(74) Representative: Sugrañes Patentes y Marcas

(56) References cited:
- WO-A2-2005/000331
- US-A1- 2004 082 655
- US-A1- 2007 078 116
- US-A1- 2008 317 805
- US-A1- 2009 098 213
- BERNARD E LEDUC ET AL: "Treatment of calcifying tendinitis of the shoulder by acetic acid iontophoresis: a double-blind randomized controlled trial", ARCHIVES OF PHYSICAL MEDICINE AND REHABILITATION, vol. 84, no. 10, 1 October 2003 (2003-10-01), pages 1523-1527, XP055062243, ISSN: 0003-9993, DOI: 10.1016/S0003-9993(03)00284-3
- PERRON ET AL: "Acetic acid iontophoresis and ultrasound for the treatment of calcifying tendinitis of the shoulder: a randomized control trial.", ARCHIVES OF PHYSICAL MEDICINE AND REHABILITATION, vol. 78, no. 4, 1 April 1997 (1997-04-01), pages 379-384, XP055062245, ISSN: 0003-9993
- DATABASE WPI Week 200538 Thomson Scientific, London, GB; AN 2005-369313 XP002696840, & JP 2005 132753 A (OGIWARA H) 26 May 2005 (2005-05-26)

## Description

### Technical Field of the Invention

The present invention relates to a pharmaceutical composition for topical administration comprising acetic acid for the treatment of calcific tendinitis and/or calcific bursitis, and more particularly to a pharmaceutical composition in the form of an emulsion especially suitable for said use.

### Background of the Invention

Calcific tendinitis/tendonitis, calcifying tendinitis or calcerous tendinitis is a common disorder characterized by deposits of hydroxyapatite (a crystalline calcium phosphate) in any tendon of the body. In most cases, these calcific (calcium) deposits tend to accumulate around the shoulder, particularly in the supraspinatus tendon, proximal bicep and elbow (epicondylitis). They can also be seen less frequently, in the heel (in the Achilles tendon), knee (in patellar tendon), wrist, fingers and forearm.

Calcific tendinitis causes chronic or acute inflammatory symptoms in patients, pain aggravated by movements and local swelling which may occur together with mobility restrictions. It has been estimated that calcific tendinitis is responsible for 7% of shoulder pain syndromes (Faure G. et al., Ann. Rheum. Dis., 42, 49-53 (1983)).

These calcium deposits can also occur in the bursa. Bursa is a sac-like structure which is located between bones, tendons and muscles having a function facilitating the movement between said structures and to reduce any friction between bones. Calcific bursitis typically leads to chronic pain, stiffness, and sometimes limited range of motion of the affected joint with use or when examined.

The conventional treatments for calcific tendinitis and calcific bursitis typically consist of preventing the condition from worsening, letting the affected part rest, administrating non-steroidal anti-inflammatory drugs (NSAIDS), physiotherapy, or injecting a steroid locally.

Other treatments intended, not only for treating the symptoms but also, to remove the calcium depositions are acetic acid iontophoresis (J. Rioja Toro et al., Rehabilitation, 35(3), 166-170 (2001) and B. E. Leduc et al., Arch Phys Med Rehabil., 84(10),1523-7 (2003)), shock wave therapy (ESWT) (described for example by MD. Po-Jung Pan et al., Arch. Phys. Med. Rehabil., 84, 988-994 (2003)) and puncturing-sucking the calcification (described for example by C. Parlier-Cuau et al., Clin. North. Am. 36, 589-96 (1998)). If the methods mentioned above are not successful, surgical treatment would be considered by means of extirpating the calcium deposition with arthroscopy or with a surgical procedure.

However, these treatments may have undesired side effects or entail certain risks. For example, frequent injections can harm the tendon and might cause or trigger a serious joint infection. Puncturing-sucking the calcification can cause inflammation and pain for a week after the puncturing. Likewise, the effectiveness of iontophoresis in treating calcifications has been strongly questioned for example by M. Perron et al., Arch. Phys. Med. Rehabil., 78(4), 379-84 (1997), B. E. Leduc et al., Arch. Phys. Med. Rehabil. 84(10), 1523-7 (2003) and C. D. Ciccone et al., Physical Therapy, 83(1), 68-74 (2003). In relation to shock wave treatment (ESWT), it is contraindicated in the chest or lung region, in patients with coagulopathies or under anti-coagulant treatments, during pregnancy, in vascular-nervous structures, in bone or target area infections, in bone or application area tumors and in growth plate (R. Sistermann et al., Z. Orthop Ihre Grenzgeb, 136, 175-81 (1998)). Additionally, adverse side effects have been described with shock wave treatments, including superficial hematomas, hyperventilations, rise in blood pressure and pain during the application thereof (M. Loew et al., J. Shoulder Elbow Sur., 4, 101-106 (1995)). Likewise, surgery and extirpation of the calcium deposit involves certain risks of complications described for example by RJ. McKendry et al., J. Rheumatol., 9, 75-80 (1982).

Based on the foregoing, there is a need to provide a new treatment for calcific tendinitis and/or calcific bursitis. In particular, the present invention provides new compositions capable of improving both the symptoms and treating calcific tendinitis and/or calcific bursitis in a definitive, less invasive and innocuous manner.

The patent document JP 2005132743 describes a dermatologic medicinal composition as an alternative for an oral administration composition. This dermatologic composition contains a pharmaceutically active ingredient glycyrrhetic acid, acetic acid and a percutaneous absorption promoter comprising a garlic ingredient that gives remarkably high percutaneous absorption effects. The dermatologic medicinal composition is effective for the treatment of onychomycosis.

### Disclosure of the Invention

The present invention relates in a broader aspect to a topically administered pharmaceutical composition comprising acetic acid for the treatment of calcific tendinitis and/or calcific bursitis and for the inflammation or pain associated with said diseases.

The compositions for topical administration of the invention have valuable therapeutic properties and are especially beneficial in the treatment of calcific tendinitis and/or calcific bursitis.

It has surprisingly been proven that after topical administration of the compositions of the present invention, patients diagnose with calcific tendinitis and/or calcific bursitis experience a quicker relief of symptoms associated with these diseases and the calcium deposits disappear after the treatment with said compositions. Therefore, the compositions for topical administration of the invention provide an easy to apply and highly effective alternative treatment of calcific tendinitis and/or calcific bursitis.

The present invention further provides stable compositions with good topical penetration and without causing any skin irritation for the local treatment of calcific tendinitis and/or calcific bursitis.

Therefore, in a first aspect, the present invention relates to a pharmaceutical composition for topical administration comprising acetic acid for the treatment of calcific tendinitis and/or calcific bursitis.

In a second aspect, the present invention also relates to a pharmaceutical composition for topical administration comprising acetic acid for the treatment, prevention or prophylaxis of inflammation or pain associated with calcific tendinitis and/or calcific bursitis.

The pharmaceutical composition described herein above can further include a percutaneous absorption promoting agent, such as for example and without limitation, urea, Aloe Vera, surfactants, azone (1-dodecylazacycloheptan-2-one), ethoxydiglycol, propylene glycol or polyethylene glycol, among others. According to the invention, the percutaneous absorption promoting agent is urea.

In a preferred embodiment of the invention, the pharmaceutical composition for topical administration comprises approximately between 17% and 22% by weight of acetic acid with respect to the total weight of the composition.

In another preferred embodiment, the pharmaceutical composition for topical administration of the present invention comprises approximately between 3% and 5% by weight of a percutaneous absorption promoting agent with respect to the total weight of the composition. Preferably, said percutaneous absorption promoting agent is urea.

Likewise, the pharmaceutical composition of the present invention can further comprise at least one compound, extract or oil with analgesic or anti-inflammatory activity, such as for example and without limitation, hydrocortisone, clobetasol, dexamethasone, prednisone, paracetamol, acetylsalicylic acid, amoxiprin, benorilate, betamethasone, choline salicylate, diflunisal, faislamine, methyl salicylate, magnesium salicylate, salsalate, diclofenac, aceclofenac, acemetacin, bromfenac, etodolac, indometacin, sulindac, tolmetin, ibuprofen, carprofen, fenbufen, fenprofen, flurbiprofen, ketoprofen, ketorolac, loxoprofen, naproxen, oxaprozin, tiaprofenic acid, suprofen, mefenamic acid, meclofenamate, meclofenamic acid, nabumetone, phenylbutazone, azapropazone, metamizole, oxyphenbutazone, sulfinpyrazone, piroxicam, lornoxicam, meloxicam, tenoxicam, celecoxib, etoricoxib, lumiracoxib, parecoxib, rofecoxib, valdecoxib, nimesulide, licofelone, oxycodone, hydrocodone, heroin, pethidine, tramadol, buprenorphine, benzocaine, lidocaine, chloroprocaine, tetracaine, procaine, tricyclic antidepressants, amitriptyline, carbamazepine, gabapentin, pregabalin, bisabolol, pantenol, biotin, disodium lauriminodipropionate tocopheryl phosphate, ciclopirox olamine, nordihydroguaiaretic acid, Arnica, madecassoside, echinacin, amaranth seed oil, sandalwood oil, peach leave extract, aloe vera extract, *Artemisia vulgaris, Asarum maximum, Calendula officinalis,* capsicum, *Centipeda cunninghamii, Chamomilla recutita, Crinum asiaticum, Hamamelis virginiana, Harpagophytum procumbens, Hypericum perforatum*, *Lilium candidum, Malva sylvestris, Melaleuca alternifolia, Origanum majorana, Salix alba, Silybum marianum, Tanacetum parthenium, Uncaria guianensis* and mixtures thereof, among others. Preferably, the compound, extract or oil with analgesic or anti-inflammatory activity is arnica or a combination of betamethasone and hydrocortisone.

Additionally, the pharmaceutical composition of the present invention can further comprise a dermatologically acceptable agent, i.e., a skin compatible agent. This dermatologically acceptable agent can preferably be presented in all pharmaceutical dosage forms normally used for topical application, for example and without limitation, in the form of an aqueous, hydroalcoholic or oily solution, an oil-in-water or water-in-oil emulsion, or multiple emulsions, an aqueous or oily gel emulsion, an emulsion of a liquid anhydrous product, an emulsion of an oil dispersion in an aqueous phase with the aid of spheroids (such as nanospheres, nanocapsules and lipid vesicles, for example), silicone in water emulsions, microemulsions, pastes, milks, balsams, foams, lotions, hydroalcoholic solutions, hydroglycolic solutions, liniment, serums, polysaccharide films, salves, fatty salves, mousses, ointments, gels, cream gels, foam gels, emulsion-gels and solutions. The preparation and composition of such dermatologically and/or cosmetically acceptable agents for topical use are already known in the art (see, for example, WO 98/00168 A1, pages 8-15 or patent US 5.681.849). Preferably, this dermatologically acceptable agent is in the form of a cream, an ointment, a salve or an emulsion.

The dermatologically acceptable agent comprised by the pharmaceutical composition of the present invention is preferably an oil-in-water-type emulsion. Preferably, said dermatologically acceptable agent in the form of an oil-in-water-type emulsion can comprises, at least and without limitation, an aqueous phase, an oily phase, emulsifiers, solvents (including water and alcohol), viscosity regulating agents, surface-active agents, dispersants, surfactants, thickeners (for example carbomers (polyacrylic acid derivatives)) or a structuring agent.

Any topically acceptable oil or lipid can be used for the oily phase (see for example, the "fatty phase constituents" cited in patent US 4,917,886, columns 4-5). The oily phase preferably comprises oily materials such as natural or synthetic oils selected from mineral oils, plant oils and animal oils, fats and waxes, fatty acid esters, fatty alcohols, fatty acids and mixtures thereof. The oily phase can also comprise active fat soluble substances for skin care.

The aqueous phase can also include water miscible organic solvents, stabilizing agents, or any water soluble or water dispersible compound compatible with an aqueous phase, such as gelling agents, film-forming polymers, thickeners, surface-active agents and the mixtures thereof.

In a preferred embodiment of the invention, the pharmaceutical composition for topical administration comprises acetic acid and a dermatologically acceptable agent. Preferably, said dermatologically acceptable agent is an emulsion. More preferably, the dermatologically acceptable agent is an oil-in-water-type emulsion.

The dermatologically acceptable agent in the form of an emulsion can additionally contain excipients, adjuvants and other dermatological additives, such as preservatives/antioxidants, fat/oil substances, water, organic solvents, silicones, thickeners, softeners, emulsifiers, anti-foaming agents, moisturizers, fragrances, surface-active agents, fillers, sequestering agents, anionic, cationic, non-ionic or amphoteric polymers or the mixtures thereof, propellants, acidifying or basifying agents, dyes, colorings, pigments or nanopigments, or any other ingredient normally formulated in dermatological or cosmetic compositions and products.

In a preferred embodiment, the pharmaceutical composition in the form of an emulsion for topical use of the present invention comprises between 30% and 55% by weight of a dermatologically acceptable agent with respect to the total weight of the composition. Said emulsion is preferably in the form of an oil-in-water emulsion.

In another preferred embodiment, the pharmaceutical composition for topical administration in the form of an emulsion of the present invention comprises acetic acid, urea and arnica. Preferably, said emulsion is in the form of an oil-in-water emulsion.

In another preferred embodiment, the pharmaceutical composition for topical administration in the form of an emulsion of the present invention comprises acetic acid, urea, hydrocortisone acetate and betamethasone. Preferably, said emulsion is in the form of an oil-in-water emulsion.

Said emulsions can, for example, be obtained by means of a method comprising dissolving the acetic acid and optionally other components, such as for example, a percutaneous absorption promoting agent and a compound, extract or oil with analgesic or anti-inflammatory activity, in water. This mixture can then be emulsified with the dermatologically acceptable agent into an emulsion. Said dermatologically acceptable agent is preferably an oil-in-water emulsion.

This composition in the form of an oil-in-water emulsion can be more or less fluid and can have the appearance of a white or colored cream, of an ointment, of a cream, of a salve, of a milk, of a lotion, of a serum, of a paste, of a foam, or of a gel. Optionally, it can be applied on skin in the form of an aerosol or by means of a patch. Likewise, it can be presented in solid form, for example in the form of a bar.

The pharmaceutical composition provided by this invention is useful for administration or application on the body of a mammal, preferably a human.

The application frequency of the pharmaceutical composition can vary greatly depending on several factors, such as the severity of the disorder and/or pathology, sex, age, weight and condition or needs of each subject. Preferably, the pharmaceutical composition for topical administration of the present invention, for example in the form of an emulsion, can be applied once, twice or three times a day.

Preferably, the pharmaceutical compositions for topical administration of the invention, for example in the form of an emulsion, can be applied on the skin for a time period between 20 days to 40 days. Preferably, the pharmaceutical compositions for topical administration in the form of an emulsion of the present invention are applied on the skin once a day for a minimum time period of 35 days.

The present invention also relates to the medical and pharmaceutical uses of a topical composition comprising acetic acid. Additionally, the present invention also relates to the medical and pharmaceutical uses of a topical composition comprising acetic acid, a percutaneous absorption promoting agent and a compound, extract or oil with analgesic and/or anti-inflammatory activity. Preferably, said topical composition is in the form of an emulsion.

In the context of the present invention, calcific bursitis and/or calcific tendinitis include, for example and without limitation: calcific tendinitis/tendonitis of the shoulder, calcific tendinitis of the supraspinatus, calcific subdeltoid bursitis, calcific subacromial bursitis, calcific prepatellar bursitis, bilateral calcific bursitis in the glenoid insertion of the long tendon of the bicep, calcific subacromiodeltoid tendinitis, calcific subacromiodeltoid bursitis, calcific tendinitis of the rotator cuff, calcifying trochanteritis of the hip, calcifying tendinitis of the sole of the foot, calcific tendinitis of the Achilles tendon and epicondilitis (tennis elbow).

The term "emulsion" refers to a mixture of immiscible liquids, where a liquid (the dispersed phase) is dispersed in another liquid (the continuous or dispersing phase). The resulting mixture has the macroscopic appearance of a single distinct phase created by means of mixing the two immiscible phases.

As used in this specification, the term "oil/water emulsion", also called a "O/W emulsion" or "oil-in-water-type emulsion", relates to emulsions in which the oily phase is dispersed in the aqueous phase, which is the continuous phase. In the present invention, the oil-in-water emulsion acts as a pharmaceutical vehicle.

As used herein, the term "active ingredient", "active substance", "pharmaceutically active substance", or "pharmaceutically active ingredient" refers to any component which potentially provides a pharmacological activity or another different effect in diagnosing, curing, alleviating, treating, or preventing a disease, or which affects the structure or function of the human body or body of other animals.

### Brief Description of the Drawings

Figure 1 shows an X-ray of a patient diagnosed with a calcification in the supraspinatus tendon before being subjected to the treatment of the present invention.
Figure 2 shows an X-ray of a patient diagnosed with a calcification in the supraspinatus tendon after being subjected to the treatment of the present invention.

### Detailed Description of the Invention

The following examples serve to illustrate the nature of the present invention. These examples are included only for illustrative purposes and must not be interpreted as limiting the invention claimed.

Two topical pharmaceutical compositions, their method of preparation and the results obtained in the treatment of calcific tendinitis and/or bursitis are described below.

### Example 1: Composition (I) in the form of a cream with hydrocortisone acetate and betamethasone.

The following table shows the different components of the composition (I) wherein the percutaneous absorption promoting agent is urea, the compounds, extracts or oils with analgesic and/or anti-inflammatory activity are hydrocortisone acetate and betamethasone, and the dermatologically and/or cosmetically acceptable agent is an oil-in-water (O/W) emulsion.

| *Materials* | % *weight* |
|---|---|
| Glacial Acetic acid | 21.04 % |
| Urea | 4.28 % |
| Hydrocortisone acetate | 3.57 % |
| Betamethasone | 0.21 % |
| O/W emulsion | 35.20 % |
| Distilled water | 35.70 % |

The O/W emulsion is used already prepared and comprises: water, isopropyl palmitate, glyceryl stearate, paraffinum liquidum, glycerin, Steareth-21, microcrystalline wax, Steareth-2, Peg-8 distearate, dimethicone, benzyl alcohol, lanolyn alcohol and phenoxy ethanol.

### Example 2: Composition (II) in the form of a cream with arnica.

In composition (II), the percutaneous absorption promoting agent is urea, the compound, extract or oil with analgesic and/or anti-inflammatory activity is arnica, and the dermatologically and/or cosmetically acceptable agent is an oil-in-water (O/W) emulsion.

| *Materials* | % *weight* |
|---|---|
| Glacial acetic acid | 21.18 % |
| Urea | 4.28 % |
| Arnica | 18.75 % |
| W/O emulsion | 52.64 % |
| Distilled water | 3.15 % |

The O/W emulsion is used already prepared and it comprises: water, isopropyl palmitate, glyceryl stearate, paraffinum liquidum, glycerin, Steareth-21, microcrystalline wax, Steareth-2, Peg-8 distearate, dimethicone, benzyl alcohol, lanolyn alcohol and phenoxy ethanol.

### Example 3: Preparation of compositions (I) and (II).

Compositions (I) and (II) are prepared according to the following method.

The amounts of urea and arnica (arnica flower glycolic extract) specified above for composition (I) or of urea and hydrocortisone acetate/betamethasone for composition (II) are added to a suitable amount of distilled water at 37°C stirring at approximately 500-1000 rpm. The mixture is left to settle in a water bath at 40°C.

Separately, the O/W emulsion is heated between 37-50°C with stirring at approximately 1100-2600 rpm for 5 minutes; after this time the previously prepared aqueous mixture is added at 40°C to the O/W emulsion stirring at 500-1000 rpm and maintaining the temperature between 37-50°C.

Finally, acetic acid is added to the cream thus prepared stirring at 500-1000 rpm and maintaining the temperature at 37-50°C.

### Results

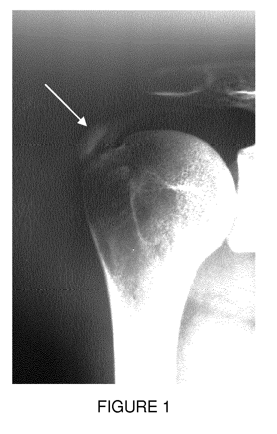

The cream of composition (II) was applied locally on a patient diagnosed with calcification in the supraspinatus tendon of the right shoulder, as shown in the X-ray of Figure 1. The cream was applied daily on the affected area for a period comprised between 30 and 40 days. Heat was locally applied by means of an electric pad for 20 minutes after each application of composition (II).

Composition (II) showed active ingredient (acetic acid) skin penetration without skin irritation.

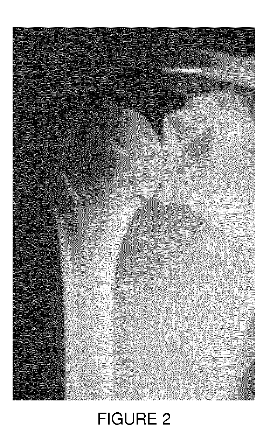

Figure 2 shows an X-ray of the same patient after the treatment with composition (II) wherein a complete disappearance of the calcification and the integrity of the tendon can be seen.

## Claims

1. A pharmaceutical composition for topical administration in the form of an emulsion comprising acetic acid and urea as a percutaneous absorption promoting agent for use in the treatment of calcific tendinitis and/or calcific bursitis.

2. A pharmaceutical composition for topical administration in the form of an emulsion comprising acetic acid and urea as a percutaneous absorption promoting agent for use in the treatment, prevention or prophylaxis of inflammation or pain associated with calcific tendinitis and/or calcific bursitis.

3. The pharmaceutical composition according to any one of claims 1 or 2 for use according to claims 1 or 2, wherein the percentage of acetic acid is between 17% and 22% by weight with respect to the total weight of the composition.

4. The pharmaceutical composition according to any one of claims 1 to 3 for use according to claims 1 or 2, wherein the percentage of percutaneous absorption promoting agent is between 3% and 5% by weight with respect to the total weight of the composition.

5. The pharmaceutical composition according to any one of claims 1 to 4 for use according to claims 1 or 2, wherein the pharmaceutical composition further comprises at least one compound, extract or oil with analgesic or anti-inflammatory activity selected from the group consisting of hydrocortisone, clobetasol, dexamethasone, prednisone, paracetamol, acetylsalicylic acid, amoxiprin, benorilate, betamethasone, choline salicylate, diflunisal, faislamine, methyl salicylate, magnesium salicylate, salsalate, diclofenac, aceclofenac, acemetacin, bromfenac, etodolac, indometacin, sulindac, tolmetin, ibuprofen, carprofen, fenbufen, fenprofen, flurbiprofen, ketoprofen, ketorolac, loxoprofen, naproxen, oxaprozin, tiaprofenic acid, suprofen, mefenamic acid, meclofenamate, meclofenamic acid, nabumetone, phenylbutazone, azapropazone, metamizole, oxyphenbutazone, sulfinpyrazone, piroxicam, lornoxicam, meloxicam, tenoxicam, celecoxib, etoricoxib, lumiracoxib, parecoxib, rofecoxib, valdecoxib, nimesulide, licofelone, oxycodone, hydrocodone, heroin, pethidine, tramadol, buprenorphine, benzocaine, lidocaine, chloroprocaine, tetracaine, procaine, tricyclic antidepressants, amitriptyline, carbamazepine, gabapentin, pregabalin, bisabolol, pantenol, biotin, disodium lauriminodipropionate tocopheryl phosphate, ciclopirox olamine, nordihydroguaiaretic acid, madecassoside, echinacin, amaranth seed oil, sandalwood oil, peach leaf extract, Aloe vera, Arnica, *Artemisia vulgaris, Asarum maximum, Calendula officinalis,* capsicum, *Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Salix alba, Silybum marianum, Tanacetum parthenium, Uncaria guianensis* and mixtures thereof.

6. The pharmaceutical composition according to claim 5 for use according to claims 1 or 2, wherein the compound, extract or oil with analgesic or anti-inflammatory activity is arnica.

7. The pharmaceutical composition according to claim 5 for use according to claims 1 or 2, wherein the compound, extract or oil with analgesic or anti-inflammatory activity is betamethasone, hydrocortisone or a mixture thereof.

8. The pharmaceutical composition according to any one of claims 1 to 7 for use according to claims 1 or 2, wherein the pharmaceutical composition further comprises a dermatologically acceptable agent selected from the group consisting of an aqueous solution, a hydroalcoholic solution, an oily solution, an oil-in-water emulsion, a water-in-oil emulsion, a multiple emulsion, an aqueous gel emulsion, an oily gel emulsion, an emulsion of a liquid anhydrous product, an emulsion of an oil dispersion in an aqueous phase with the aid of spheroids, a silicone in water emulsion, microemulsions, pastes, milks, balsams, foams, lotions, hydroalcoholic solutions, hydroglycolic solutions, liniment, serums, polysaccharide films, salves, fatty salves, mousses, ointments, gels, cream gels, foam gels, an emulsion-gels and solutions.

9. The pharmaceutical composition according to claim 8 for use according to claims 1 or 2, wherein the dermatologically acceptable agent is an oil-in-water emulsion.

10. The pharmaceutical composition according to claim 8 or 9 for use according to claims 1 or 2, wherein the percentage of dermatologically acceptable agent is between 30% and 55% by weight with respect to the total weight of the composition.

11. A cream comprising the pharmaceutical composition defined in any one of claims 1 to 11 for use according to claims 1 or 2.

## Patentansprüche

1. Pharmazeutische Zusammensetzung zur topischen Verabreichung in Form einer Emulsion umfassend Essigsäure und Harnstoff als die perkutane Absorption förderndes Mittel für deren Verwendung bei der Behandlung von kalzifizierender Tendinitis und/oder kalzifizierender Bursitis.

2. Pharmazeutische Zusammensetzung zur topischen Verabreichung in Form einer Emulsion umfassend Essigsäure und Harnstoff als die perkutane Absorption förderndes Mittel für deren Verwendung bei der Behandlung, Vorbeugung oder Prophylaxe von Entzündung oder Schmerz assoziiert mit kalzifizierender Tendinitis und/oder kalzifizierender Bursitis.

3. Pharmazeutische Zusammensetzung nach einem der Ansprüche 1 oder 2 für deren Verwendung nach den Ansprüchen 1 oder 2, wobei der Anteil von Essigsäure zwischen 17 Gew.-% und 22 Gew.-% in Bezug auf das Gesamtgewicht der Zusammensetzung liegt.

4. Pharmazeutische Zusammensetzung nach einem der Ansprüche 1 bis 3 für deren Verwendung nach den Ansprüchen 1 oder 2, wobei der Anteil vom die perkutane Absorption fördernden Mittel zwischen 3% und 5 Gew.-% in Bezug auf das Gesamtgewicht der Zusammensetzung liegt.

5. Pharmazeutische Zusammensetzung nach einem der Ansprüche 1 bis 4 für deren Verwendung nach den Ansprüchen 1 oder 2, wobei die pharmazeutische Zusammensetzung zusätzlich mindestens eine Verbindung, einen Extrakt oder ein Öl mit schmerzlindernder oder entzündungshemmender Aktivität umfasst, ausgewählt aus der Gruppe bestehend aus Hydrocortison, Clobetasol, Dexamethason, Prednison, Paracetamol, Acetylsalicylsäure, Amoxiprin, Benorilat, Betamethason, Cholinsalicylat, Diflunisal, Faislamin, Methylsalicylat, Magnesiumsalicylat, Salsalat, Diclofenac, Aceclofenac, Acemetacin, Bromfenac, Etodolac, Indometacin, Sulindac, Tolmetin, Ibuprofen, Carprofen, Fenbufen, Fenprofen, Flurbiprofen, Ketoprofen, Ketorolac, Loxoprofen, Naproxen, Oxaprozin, Tiaprofensäure, Suprofen, Mefenaminsäure, Meclofenamat, Meclofenaminsäure, Nabumeton, Phenylbutazon, Azapropazon, Metamizol, Oxyphenbutazon, Sulfinpyrazon, Piroxicam, Lornoxicam, Meloxicam, Tenoxicam, Celecoxib, Etoricoxib, Lumiracoxib, Parecoxib, Rofecoxib, Valdecoxib, Nimesulid, Licofelon, Oxycodon, Hydrocodon, Heroin, Pethidin, Tramadol, Buprenorphin, Benzocain, Lidocain, Chlorprocain, Tetracain, Procain, trizyklischen Antidepressiva, Amitriptylin, Carbamazepin, Gabapentin, Pregabalin, Bisabolol, Pantenol, Biotin, Dinatriumlauriminodipropionat-Tocopherylphosphate, Ciclopiroxolamin, Nordihydroguajaretsäure, Madecassosid, Echinacin, Amaranthsamenöl, Sandelholzöl, Pfirsichblattextrakt, Aloe vera, *Arnica, Artemisia vulgaris, Asarum maximum, Calendula officinalis, Capsicum, Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Salix alba, Silybum marianum, Tanacetum parthenium, Uncaria guianensis* und Mischungen derselben.

6. Pharmazeutische Zusammensetzung nach Anspruch 5 für deren Verwendung nach den Ansprüchen 1 oder 2, wobei die Verbindung, der Extrakt oder das Öl mit schmerzlindernder oder entzündungshemmender Aktivität *Arnica* ist.

7. Pharmazeutische Zusammensetzung nach Anspruch 5 für deren Verwendung nach den Ansprüchen 1 oder 2, wobei die Verbindung, der Extrakt oder das Öl mit schmerzlindernder oder entzündungshemmender Aktivität Betamethason, Hydrocortison oder eine Mischung derselben ist.

8. Pharmazeutische Zusammensetzung nach einem der Ansprüche 1 bis 7 für deren Verwendung nach den Ansprüchen 1 oder 2, wobei die pharmazeutische Zusammensetzung zusätzlich ein dermatologisch akzeptables Mittel umfasst, ausgewählt aus der Gruppe bestehend aus einer wässrigen Lösung, einer hydroalkoholischen Lösung, einer öligen Lösung, einer Öl-in-Wasser-Emulsion, einer Wasser-in-Öl-Emulsion, einer multiplen Emulsion, einer wässrigen Gelemulsion, einer öligen Gelemulsion, einer Emulsion eines flüssigen wasserfreien Produkts, einer Emulsion einer Öldispergierung in einer wässrigen Fase mithilfe von Sphäroiden, einer Silicon-in-Wasser-Emulsion, Mikroemulsionen, Pasten, Milchen, Balsamen, Schäumen, Lotionen, hydroalkoholischen Lösungen, hydroglycolischen Lösungen, Einreibemitteln, Sera, Polysaccharid-Filmen, Salben, fettigen Salben, Haarschäumen, Wundsalben, Gelen, Cremegelen, Schaumgelen, Emulsionsgelen und Lösungen.

9. Pharmazeutische Zusammensetzung nach Anspruch 8 für deren Verwendung nach den Ansprüchen 1 oder 2, wobei das dermatologisch akzeptable Mittel eine Öl-in-Wasser-Emulsion ist.

10. Pharmazeutische Zusammensetzung nach Anspruch 8 oder 9 für deren Verwendung nach den Ansprüchen 1 oder 2, wobei der Anteil vom dermatologisch akzeptablen Mittel zwischen 30 Gew.-% und 55 Gew.-% in Bezug auf das Gesamtgewicht der Zusammensetzung liegt.

11. Creme umfassend die in einer der Ansprüche 1 bis 11 definierte pharmazeutische Zusammensetzung für deren Verwendung nach den Ansprüchen 1 oder 2.

## Revendications

1. Composition pharmaceutique pour administration topique sous la forme d'une émulsion comprenant de l'acide acétique et de l'urée comme agent promoteur d'absorption percutanée pour l'utilisation dans le traitement de la tendinite calcifiée et/ou la bursite calcifiée.

2. Composition pharmaceutique pour administration topique sous la forme d'une émulsion comprenant de l'acide acétique et de l'urée comme agent promoteur d'absorption percutanée pour l'utilisation dans le traitement, la prévention ou la prophylaxie de l'inflammation ou de la douleur associée à la tendinite calcifiée et/ou la bursite calcifiée.

3. Composition pharmaceutique selon l'une quelconque des revendications 1 ou 2 pour l'utilisation selon les revendications 1 ou 2, où le pourcentage d'acide acétique est compris entre 17% et 22 % en poids par rapport au poids total de la composition.

4. Composition pharmaceutique selon l'une quelconque des revendications 1 à 3 pour l'utilisation selon les revendications 1 ou 2, où le pourcentage d'agent promoteur d'absorption percutanée est compris entre 3 % et 5 % en poids par rapport au poids total de la composition.

5. Composition pharmaceutique selon l'une quelconque des revendications 1 à 4 pour l'utilisation selon les revendications 1 ou 2, où la composition pharmaceutique comprend en outre au moins un composé, un extrait ou une huile avec activité analgésique ou anti-inflammatoire choisi dans le groupe constitué de : hydrocortisone, clobétasol, dexaméthasone, prednisone, paracétamol, acide acétylsalicylique, amoxiprine, bénorilate, bétaméthasone, salicylate de choline, diflunisal, faislamine, salicylate de méthyle, salicylate de magnésium, salsalate, diclofénac, acéclofénac, acémétacine, bromfénac, étodolac, indométacine, sulindac, tolmétine, ibuprofène, carprofène, fenbufène, fenprofène, flurbiprofène, kétoprofène, kétorolac, loxoprofène, naproxène, oxaprozine, acide tiaprofénique, suprofène, acide méfénamique, méclofénamate, acide méclofénamique, nabumétone, phénylbutazone, azapropazone, métamizole, oxyphenbutazone, sulfinpyrazone, piroxicam, lornoxicam, méloxicam, ténoxicam, célécoxib, étorcoxib, lumiracoxib, parécoxib, rofécoxib, valdécoxib, nimesulide, licofélone, oxycodone, hydrocodone, héroïne, péthidine, tramadol, buprenorphine, benzocaïne, lidocaïne, chloroprocaïne, tétracaïne, procaïne, antidépresseurs tricycliques, amitriptyline, carbamazépine, gabapentine, prégabaline, bisabolol, panténol, biotine, lauriminodipropionate de disodium-phosphate de tocophéryle, ciclopirox olamine, acide nordihydroguaïarétique, madécassoside, échinacine, huile de graine d'amaranthe, huile de bois de santal, extrait de feuille de pêcher, Aloe vera, *Arnica, Artemisia vulgaris, Asarum maximum, Calendula officinalis, Capsicum, Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Salix alba, Silybum marianum, Tanacetum parthenium, Uncaria guianensis* et des mélanges de ceux-ci.

6. Composition pharmaceutique selon la revendication 5 pour l'utilisation selon les revendications 1 ou 2, où le composé, l'extrait ou l'huile avec activité analgésique ou anti-inflammatoire est de l'*Arnica*.

7. Composition pharmaceutique selon la revendication 5 pour l'utilisation selon les revendications 1 ou 2, où le composé, l'extrait ou l'huile avec activité analgésique ou anti-inflammatoire est de la bétaméthasone, de l'hydrocortisone ou un mélange de celles-ci.

8. Composition pharmaceutique selon l'une quelconque des revendications 1 à 7 pour l'utilisation selon les revendications 1 ou 2, où la composition pharmaceutique comprend en outre un agent dermatologiquement acceptable choisi dans le groupe constitué de : une solution aqueuse, une solution hydroalcoolique, une solution huileuse, une émulsion huile-dans-eau, une émulsion eau-dans-huile, une émulsion multiple, une émulsion de gel aqueuse, une émulsion de gel huileuse, une émulsion d'un produit anhydre liquide, une émulsion d'une dispersion d'huile dans une phase aqueuse à l'aide de sphéroïdes, une émulsion de silicone dans l'eau, des microémulsions, des pâtes, des laits, des baumes, des mousses, des lotions, des solutions hydroalcooliques, des solutions hydroglycoliques, des liniments, des sérums, des films de polysaccharide, des onguents, des onguents gras, des mousses coiffantes, des pommades, des gels, des gels crème, des gels mousse, des gels émulsion et des solutions.

9. Composition pharmaceutique selon la revendication 8 pour l'utilisation selon les revendications 1 ou 2, où l'agent dermatologiquement acceptable est une émulsion huile-dans-eau.

10. Composition pharmaceutique selon les revendications 8 ou 9 pour l'utilisation selon les revendications 1 ou 2, où le pourcentage d'agent dermatologiquement acceptable est compris entre 30 % et 55 % en poids par rapport au poids total de la composition.

11. Crème comprenant la composition pharmaceutique selon l'une quelconque des revendications 1 à 11 pour l'utilisation selon les revendications 1 ou 2.
